# EUROPÄISCHE PATENTSCHRIFT

(11) **EP 4 346 643 B1**
(45) Veröffentlichungstag und Bekanntmachung des Hinweises auf die Patenterteilung: **15.04.2026**
(21) Anmeldenummer: 22727261.4
(22) Anmeldetag: 25.05.2022
(51) Int. Cl.: A61B 17/16

(54) **ZWISCHENWIRBELPRÄPARATIONSWERKZEUG**
INTERVERTEBRAL-PREPARATION TOOL
INSTRUMENT DE PRÉPARATION INTERVERTÉBRALE

(30) Priorität: 28.05.2021 DE 102021205450
(43) Veröffentlichungstag der Anmeldung: 10.04.2024
(73) Patentinhaber: Richard Wolf GmbH, 75438 Knittlingen (DE)
(72) Erfinder: MAUL, Silas, 75180 Pforzheim (DE); FREY, Sebastian, 68753 Waghäusel (DE)
(74) Vertreter: Patentanwälte Hemmer Lindfeld Frese Partnerschaft mbB
(86) Internationale Anmeldenummer: PCT/DE2022/200104
(87) Internationale Veröffentlichungsnummer: WO 2022/247998

(56) Entgegenhaltungen:
- WO-A1-2008/035849
- US-A1- 2019 038 304
- US-B1- 6 224 604

## Beschreibung

Die Erfindung betrifft ein Zwischenwirbelpräparationswerkzeug, d.h. ein Abtragwerkzeug für die Zwischenwirbelpräparation, einen so genannten Paddle Shaver.

Zur Zwischenwirbelpräparation, d.h. zum Abtragen der Bandscheibe zwischen den Wirbeln vor dem Einsetzen eines Implantats sind spezielle Abtragwerkzeuge, so genannte Paddle Shaver bekannt. Beispielsweise WO 2008/035849 A1 offenbart ein solches Instrument, welches an seinem distalen Ende einen Schneidkopf mit zwei Klingen aufweist. Die Klingen sind zur Längsachse des Schneidkopfes diametral entgegengesetzt gerichtet und über eine Betätigungsstange bewegbar. Dazu sind die Klingen jeweils über zwei Hebel gelenkig angelenkt und werden durch ein Federelement nach außen gedrückt. Die zwei Hebel ermöglichen ein Verkippen der Klingen zur Längsachse des Instrumentes. Ein solches Verkippen ist im Einzelfall unerwünscht.

US 6,224,604 B1 offenbart einen aufweitbaren orthopädischen Bohrer, welcher bei Operationen zur Wirbelsäulenversteifung angewandt werden kann. Der Bohrer weist außenliegende Schneidelemente auf, welche über eine Hebelmechanik aufweitbar sind. Diese Hebelmechanik weist zwei Paare von Hebeln auf, welche jeweils gelenkig an einem gemeinsamen Anlenkungspunkt an dem Schneidelement angelenkt sind. Von dem ersten Hebelpaar ist ein erster Hebel an einer äußeren Hülse des Bohrers festgelegt und ein zweiter Hebel ist gemeinsam mit dem ersten Hebel des zweiten Hebelpaars an einer beweglichen Hülse festgelegt. Die bewegliche Hülse gleitet auf einen zentralen Schaft. Am distalen Ende des zentralen Schaftes ist der zweite Hebel des zweiten Hebelpaars gelenkig angelenkt. Auf diese Weise kann durch Bewegung des Schaftes in der Hülse das Schneidelement radial nach außen bewegt werden, wobei es parallel zu der Längsachse des Schaftes ausgerichtet bleibt. Diese Anordnung benötigt relativ viel Raum für die zwei Hebelpaare und die bewegliche Hülse, sodass der minimale Durchmesser des Instrumentes recht groß ist.

Es ist Aufgabe der Erfindung, ein verbessertes Abtragwerkzeug für die Zwischenwirbelpräparation, das heißt einen Paddle Shaver, bereitzustellen, welcher eine definierte Führung der Abtrag-Klingen bei einem kleinen minimalen Durchmesser gewährleistet.

Diese Aufgabe wird durch ein Zwischenwirbelpräparationswerkzeug, d.h. ein Abtragwerkzeug für die Zwischenwirbelpräparation mit den in Anspruch 1 angegebenen Merkmalen gelöst. Bevorzugte Ausführungsformen ergeben sich aus den Unteransprüchen, der nachfolgenden Beschreibung sowie den beigefügten Figuren.

Das erfindungsgemäße Zwischenwirbelpräparationswerkzeug ist ausgebildet zum Einsatz als Abtragwerkzeug für die Zwischenwirbelpräparation, d.h. ein so genannter Paddle Shaver, und weist einen Schneidkopf mit zumindest einer beweglichen Klinge auf. Die Klinge ist insbesondere beweglich, um sie nach dem Einführen in den Zwischenwirbelraum, d.h. zwischen zwei Wirbel in eine bezüglich der Längsachse radial beabstandete Position bewegen zu können, um Bandscheibenmaterial im gesamten Zwischenwirbelbereich abtragen zu können. Die zumindest eine bewegliche Klinge ist von zumindest drei schwenkbaren Hebeln gehalten. D.h. die schwenkbaren Hebel verbinden die Klinge mit dem Schneidkopf bzw. der tragenden Struktur des Schneidkopfes und stellen die gewünschte Beweglichkeit der Klinge her. Die Hebel selber sind starr ausgebildet. Ein erster und ein zweiter Hebel sind jeweils an einem ersten Anlenkungspunkt an der Klinge und an einem beabstandeten zweiten Anlekungspunkt an einem Tragelement bzw. einer Tragstruktur des Schneidkopfes angelenkt, d.h. schwenkbar verbunden. Der erste und der zweite Anlenkungspunkt liegen an den Hebeln bevorzugt an deren entgegengesetzten Enden. Ein dritter Hebel ist an einem ersten Anlenkungspunkt an der Klinge und an einem beabstandeten zweiten Anlenkungspunkt an einer Betätigungsstange angelenkt, d.h. schwenkbar verbunden. Diese Betätigungsstange ist relativ zu dem Tragelement verschiebbar. Der erste und der zweite Anlenkungspunkt an dem dritten Hebel liegen ebenfalls bevorzugt an den entgegengesetzten Enden dieses Hebels. Durch Verschieben der Betätigungsstange kann der dritte Hebel somit relativ zu dem Tragelement bewegt werden, wodurch ein Bewegen der Klinge bzw. Spreizen des Instrumentes im Bereich des Schneidkopfes zur Durchmessererweiterung möglich ist. Die Anlenkungspunkte sind dazu als Schwenkgelenke ausgebildet, deren Schwenkachsen sich parallel zueinander erstrecken. So ist insbesondere ein Verschwenken der Hebel möglich, welches eine Bewegung der Schneidklinge in radialer Richtung bezogen auf die Längsachse des Instrumentes bzw. des Schneidkopfes bewirkt, um den Durchmesser zu erweitern. Die Anordnung von drei Hebeln, welche die Klinge an dem Schneidkopf tragen, hat den Vorteil, dass ein Verkippen der Klinge in der Weise, dass eine Veränderung der Winkellage zur Längsachse des Schneidkopfes, welche sich vom proximalen zum distalen Ende erstreckt, nicht mehr möglich ist und somit die Klinge in einer definierten Winkellage gehalten wird. Die Winkellage relativ zur Längsachse kann konstant sein oder sich mit der Bewegung des dritten Hebels definiert ändern. Ein undefiniertes Verkippen der Klinge wird jedoch durch die Verwendung von drei Hebeln verhindert.

Bevorzugt weist das Abtragwerkzeug eine zweite Klinge auf. Diese Klinge kann starr sein. Bevorzugt weist der Schneidkopf jedoch zwei bewegliche Klingen auf, wobei diese zwei Klingen jeweils von drei schwenkbaren Hebeln gehalten sind, wie sie vorangehend beschrieben wurden. D.h. jede der zwei Klingen ist über drei starr ausgebildeten Hebel an dem Schneidkopf gehalten, wobei jeweils ein erster und ein zweiter Hebel jeweils an einem Anlenkungspunkt an der zugehörigen Klinge und einem beabstandeten zweiten Anlenkungspunkt an dem Tragelement angelenkt sind. Dabei können die ersten und zweiten Hebel der beiden Klingen an separaten zweiten Anlenkungspunkten an dem Tragelement angelenkt sein. Es wäre jedoch auch denkbar, dass die beiden ersten Hebel an einem gemeinsamen zweiten Anlenkungspunkt und/oder die beide zweiten Hebel an einem gemeinsamen zweiten Anlenkungspunkt an dem Tragelement schwenkbar angelenkt sind. Ferner weist jede der Klingen einen dritten Hebel auf, welcher an einem ersten Anlenkungspunkt an der zugehörigen Klinge und an einem beabstandeten zweiten Anlenkungspunkt an der relativ zu dem Tragelement verschiebbaren Betätigungsstange angelenkt ist. Die Anlenkungspunkte sind als Schwenkgelenke mit zueinander parallelen Schwenkachsen ausgebildet, wobei insbesondere auch die Schwenkachsen der Anlenkungspunkte beider Klingen parallel zueinander verlaufen. Auch für die beiden dritten Hebel könnte ein gemeinsamer zweiter Anlenkungspunkt an der Betätigungsstange vorgesehen sein. Ferner könnten die dritten Hebel an einer Klinge jeweils an einem gemeinsamen ersten Anlenkungspunkt mit einem zweiten Hebel angelenkt sein.

Die zumindest eine Klinge oder die Klingen sind zweckmäßigerweise so ausgebildet, dass ihre Längskanten, d.h. ihre langen Kanten, welche sich vorzugsweise im Wesentlichen in Instrumentenlängsrichtung erstrecken, schneidend ausgebildet sind bzw. als Schneidkanten wirken. Die Klingen wirken somit in Umfangsrichtung bezogen auf die Instrumenten- bzw. Werkzeuglängsachse schneidend bzw. abtragend. So kann das Werkzeug in einer Weise eingesetzt werden, dass durch eine Bewegung quer zur Instrumentenlängsachse und/oder Drehung um die Instrumentenlängsachse ein Schneiden oder Abtragen bewirkt werden kann.

Die zwei dritten Hebel bilden bevorzugt eine Kniehebelmechanik, welche durch Verschieben der Betätigungsstange die beiden Klingen auseinander drückt bzw. in umgekehrter Richtung zusammenzieht, sodass das Instrument im Bereich des Schneidkopfes in Durchmesserrichtung aufgeweitet oder verkleinert werden kann, wobei der Durchmesser durch den radialen Abstand der Klingen definiert wird. Durch die Anordnung der drei Hebel an jeder Klinge wird dabei die Winkellage der beiden Klingen zueinander fest vorgegeben, d.h. die beiden Klingen können nicht undefiniert zueinander verschwenken oder ihre Winkellage zueinander ändern.

Vorzugsweise sind die zwei Klingen an entgegengesetzten Seiten einer Längsachse des Schneidkopfes angeordnet. Die Längsachse des Schneidkopfes ist dabei die sich vom proximalen zum distalen Ende hin erstreckende Achse. Die Klingen sind weiter bevorzugt an diametral entgegengesetzten Seiten bezüglich der Längsachse angeordnet, sodass die Klingen bzw. der Abstand der Außenseiten der Klingen zueinander den Durchmesser des Schneidkopfes definiert. Durch Bewegen einer Klinge oder beider Klingen in Richtung quer zur Längsachse, insbesondere in radialer Richtung, kann der Durchmesser bzw. der Abstand der Klingen zueinander verändert werden. Weiter bevorzugt sind die beiden Klingen symmetrisch zueinander angeordnet, d.h. insbesondere spiegelbildlich bezüglich der Längsachse bzw. einer Ebene, in welcher die Längsachse gelegen ist. Insbesondere wenn beide Klingen bewegbar sind, werden die Klingen bevorzugt symmetrisch zueinander von der Längsachse weg oder zu der Längsachse hin bewegt, was wie oben beschrieben über die dritten Hebel, welche dann eine Art Kniehebelgelenk bilden, realisiert wird.

Die zweiten Anlenkungspunkte der drei Hebel zum Halten einer Klinge, d.h. einer einzelnen Klinge, sind vorzugsweise in einer Richtung quer zu den Schwenkachsen voneinander beabstandet. Wenn zwei Klingen vorgesehen sind, sind weiter bevorzugt die zweiten Anlenkungspunkte der drei Hebel beider Klingen in Richtung quer zu den Schwenkachsen voneinander beabstandet. Die Richtung quer zu den Schwenkachsen ist vorzugsweise eine Richtung parallel zur Längsachse des Schneidkopfes. Die Schwenkachsen erstrecken sich vorzugsweise quer, insbesondere rechtwinklig zu der Längsachse des Schneidkopfes. Durch die beabstandete Anordnung der Anlenkungspunkte wird eine große Stabilität erreicht. Beispielsweise könnten der erste und der zweite Hebel gemeinsam mit der Klinge und dem Tragelement eine Parallelogrammstruktur bilden. Ein dritter Hebel erstreckt sich vorzugsweise nicht parallel, sondern gewinkelt zu den anderen beiden Hebeln, sodass das Verkippen verhindern und eine Kraftübertragung in allen Richtungen möglich wird. Bevorzugt erstrecken sich zumindest zwei Hebel in dem Zustand, in dem die Klinge radial ausgefahren ist, nicht parallel zueinander. Der radial ausgefahrene Zustand ist ein Zustand, in dem die Klinge in radialer Richtung weiter von dem Tragelement bzw. der Längsachse des Schneidkopfes beabstandet ist als in ihrer Ruhelage. In der Ruhelage befindet sich die Klinge in der Position, in welcher sie den geringsten Abstand zur Längsachse des Schneidkopfes hat und vorzugsweise direkt an dem Tragelement anliegt. In diesem Ruhezustand können sich alle drei Hebel, welche eine Klinge tragen, im Wesentlichen parallel zueinander oder entlang einer Achse erstrecken, sodass insgesamt der Durchmesser des Instrumentes in diesem Zustand minimiert ist.

Weiter bevorzugt bildet am Schneidkopf der erste Hebel einen distalseitigen Hebel und der dritte Hebel bildet einen proximalseitigen Hebel, wobei der zweite Hebel zwischen dem ersten und dem dritten Hebel angeordnet ist. Der distalseitige Hebel ist derjenige Hebel, welcher am weitesten zum distalen Ende des Schneidkopfes hin gelegen ist, während der proximale Hebel derjenige Hebel ist, welcher am weitesten zum proximalen Ende hin gelegen ist. So sind die drei Hebel für jede vorhandene Klinge, welche über drei Hebel angelenkt ist, in Richtung der Längsachse des Schneidkopfes versetzt zueinander angeordnet. Dabei sind die Hebel vorzugsweise so weit versetzt, dass sie im zusammengeklappten Zustand, in dem sich die Schneidklinge in ihrer Ruhelage und der radial am weitesten innenliegenden Position befindet, in Längsrichtung vollständig hintereinander gelegen sind. So kann ein möglichst kleiner Instrumentendurchmesser im zusammengeklappten bzw. eingefahrenen Zustand erreicht werden. D.h. in diesem Zustand liegt vorzugsweise der zweite Anlenkungspunkt des ersten Hebels am weitesten distalwärts und der erste Anlenkungspunkt des ersten Hebels liegt weiter distalwärts als der zweite Anlenkungspunkt des zweiten Hebels. Der erste Anlenkungspunkt des zweiten Hebels liegt wiederum weiter distalwärts als der dritte Hebel, insbesondere als der erste Anlenkungspunkt des dritten Hebels. Besonders bevorzugt liegt der zweite Anlenkungspunkt des dritten Hebels an der Betätigungsstange am weitesten proximalwärts.

Gemäß einer weiteren bevorzugten Ausführungsform sind an einer Klinge die ersten Anlenkungspunkte des ersten und des zweiten Hebels und vorzugsweise die ersten Anlenkungspunkte des ersten, des zweiten und des dritten Hebels in einer Richtung quer zu den Schwenkachsen, d.h. vorzugsweise parallel zur Längsrichtung des Schneidkopfes, voneinander beabstandet. Dies ermöglicht zum einen eine Parallelogrammstruktur, wie sie oben beschrieben wurde. Unabhängig von dieser kann zum anderen eine gleichmäßige Kraftübertragung zwischen Klinge und Trägerelement erreicht werden, welche insbesondere ein Verkippen der Klinge verhindert. Ferner ermöglicht diese Anordnung zusammen mit den Abständen der zweiten Anlenkungspunkte und entsprechender Hebellängen eine Ruhelange, in der die Hebel in der vorangehend beschriebenen Weise in axialer Richtung hintereinander liegen. Wenn zwei Klingen jeweils über drei Hebel beweglich an dem Trägerelement angeordnet sind, sind die Anlenkungspunkte vorzugsweise an beiden Klingen in entsprechender Weise angeordnet, vorzugsweise in der oben beschriebenen Weise symmetrisch zur Längsachse bzw. einer sich entlang der Längsachse erstreckenden Ebene.

Die Hebel und deren Anlenkungspunkte sind weiter bevorzugt so gelegen und dimensioniert, dass durch Verschwenken der Hebel um ihre zweiten Anlenkungspunkte die zugehörige Klinge von einer ersten dem Tragelement nahen in zumindest eine zweite von dem Tragelement weiter beabstandete Position bewegbar ist. Die dem Tragelement nahe Position ist dabei die vorangehend genannte Ruhelage. Beim Verschwenken drehen sich die Hebel um ihre zweiten Anlenkungspunkte an dem Trägerelement, sodass sich der Abstand der ersten Anlenkungspunkte an der Klinge in radialer Richtung zu der Längsachse des Tragelementes vergrößert und die Klingen in radialer Richtung ausgefahren bzw. von dem Tragelement und der Längsachse wegbewegt werden.

Vorzugsweise sind der erste und der zweite Hebel so angelenkt, dass sie zum Bewegen der verbundenen Klinge in derselben Drehrichtung verschwenken. Bevorzugt verschwenken die Hebel dabei so, dass sich die ersten Anlenkungspunkte mit der Klinge neben der Bewegung in radialer Richtung gleichzeitig in Längsrichtung distalwärts bewegen. Dies kann beispielsweise nach Art einer Parallelogrammführung geschehen, wenn die Hebel unterschiedlich lang ausgebildet sind, kann dabei jedoch gleichzeitig eine definierte Schräglage der Klinge erreicht werden, welche von der genauen Parallelogrammführung abweicht.

Der dritte Hebel ist weiter bevorzugt so angelenkt, dass er zum Bewegen der verbundenen Klinge in eine Drehrichtung verschwenkt, welche der Drehrichtung des ersten und/oder zweiten Hebels bei deren gleichzeitigen Verschwenken entgegengesetzt ist. D.h. der dritte Hebel kann sich beispielsweise so bewegen, dass relativ zu der zweiten Anlenkungspunkt der erste Anlenkungspunkt des dritten Hebels an der Klinge in proximaler Richtung verschwenkt. Durch die Bewegung der Betätigungsstange bewegt sich jedoch vorzugsweise gleichzeitig der zweite Anlenkungspunkt in distaler Richtung, sodass insgesamt der gesamte dritte Hebel relativ zum Tragelement seine Position beibehält oder in distaler Richtung bewegt wird. Durch die Schwenkbewegung in entgegengesetzter Richtung kann erreicht werden, dass sich der dritte Hebel gewinkelt zu dem zweiten Hebel und vorzugsweise dem ersten und dem zweiten Hebel erstreckt, sodass alle drei Hebel gemeinsam keine Parallelogrammanordnung bilden und so ein Verkippen der Klinge verhindert wird. D.h. der zweite und der dritte Hebel erstrecken sich vorzugsweise entgegengesetzt gewinkelt zum Radius bezogen auf die Längsachse. Wenn zwei Klingen vorgesehen sind, werden deren dritte Hebel, wie oben beschrieben, über die Betätigungsstange vorzugsweise nach Art eines Kniehebelgelenks bewegt, sodass durch die axiale Verschiebung der Betätigungsstange die ersten Anlenkungspunkte der dritten Hebel radial nach außen gedrückt werden, sodass die beiden Klingen auseinander bewegt werden. In umgekehrter Richtung können die Klingen entsprechend wieder zusammenbewegt, d.h. zurück in eine an dem Tragelement anliegende Ruhelage bewegt werden.

Der erste und der zweite Hebel, welche an einer Klinge, d.h. an derselben Klinge angelenkt sind, können zwischen ihren ersten und zweiten Anlenkungspunkten dieselbe Länge aufweisen. Wenn gleichzeitig der Abstand der ersten Anlenkungspunkte voneinander und der Abstand zwischen den zweiten Anlenkungspunkten gleich ist, wird somit im Wesentlichen eine Parallelogrammform bzw. Parallelogrammanordnung geschaffen, wie sie vorangehend beschrieben wurde.

Ferner ist es möglich, dass der zweite und der dritte Hebel, welche an eine Klinge angelenkt sind, zwischen ihren ersten und zweiten Anlenkungspunkten dieselbe Länge aufweisen. Dies bewirkt, dass der zweite und der dritte Hebel beim Verschwenken eine Dreiecks- oder Trapezform zueinander definieren, welche insbesondere eine Kraftübertragung sowohl in distaler als auch in proximaler Richtung zwischen der Klinge und dem Tragelement bzw. der Betätigungsstange ermöglicht. Diese Ausgestaltung verhindert das Verkippen der Klinge, sodass verhindert wird, dass sich die Winkellage der Klinge relativ zu der Längsachse des Schneidkopfes undefiniert ändert.

In einer speziellen Ausführungsform können der erste Hebel und der zweite Hebel, welche an einer Klinge, d.h. an derselben Klinge angelenkt sind, zwischen ihren ersten und zweiten Anlenkungspunkten unterschiedlichen Längen aufweisen, wobei z.B. der erste Hebel, welcher einen distalseitigen Hebel am Schneidkopf bildet, länger als der zweite Hebel ausgebildet ist. Alternativ könnte auch der erste Hebel kürzer als der zweite Hebel sein. Hierdurch kann erreicht werden, dass beim radialen Bewegen der Klinge nach außen durch Verschwenken des dritten Hebels sich gleichzeitig die Winkellage der Klinge relativ zur Längsachse der Schneidkopfes ändert. D.h. die Klinge bewegt sich nicht parallel in radialer Richtung, sondern verschwenkt bei der radialen Bewegung gleichzeitig um ein gewisses Maß. So bildet sich ein Lordosewinkel, welcher bei bestimmten Einsatzzwecken erwünscht sein kann.

Der zweite und der dritte Hebel, welche an einer Klinge, d.h. an derselben Klinge angelenkt sind, können zwischen ihren ersten und zweiten Anlenkungspunkten unterschiedliche Längen aufweisen, wobei z.B. der zweite Hebel länger als der dritte Hebel ausgebildet ist. Eine andere Länge für den dritten Hebel kann Vorteile im Hinblick auf die Funktion des dritten Hebels für die Ausstellbewegung bzw. radiale Bewegung der Klinge haben. Ferner kann es von Vorteil sein, den dritten Hebel kürzer auszubilden, falls der erste Hebel länger ist, um einen gewünschten Lordosewinkel, d.h. eine konische Form des Schneidkopfes realisieren zu können. Umgekehrt könnte der dritte Hebel länger sein, falls z.B. der erste Hebel kürzer ist.

Vorzugsweise ist der Schneidkopf am distalen Ende eines Instrumentenschaftes gelegen und die Betätigungsstange ist in Längsrichtung des Instrumentenschaftes an oder in diesem verschiebbar geführt. So wird am distalen Ende eine Relativbewegung zwischen Betätigungsstange und dem Instrumentenschaft bzw. einem fest mit dem Instrumentenschaft verbundenen Tragelement realisiert. Durch diese Relativbewegung wird der zweite Anlenkungspunkt des dritten Hebels verlagert, sodass dieser Hebel dann in Zusammenwirkung mit dem ersten und zweiten Hebel dazu führt, dass die zugehörige Schneidklinge radial bezogen auf die Längsachse nach außen verlagert wird und der Schneidkopf aufgeweitet wird. Vorzugsweise wird das Aufweiten durch Vorschieben der Betätigungsstange in distaler Richtung und das Wiederzusammenziehen des Schneidkopfes durch umgekehrte Bewegung der Betätigungsstange in proximaler Richtung erreicht.

Der Schneidkopf ist vorzugsweise so ausgebildet, dass er, wenn die Klinge oder die Klingen in ihrer anliegenden Ruhelage sind, am distalen Ende eine stumpfe Spitze aufweist, welche vorzugsweise direkt an dem Tragelement angebracht ist oder von dem Tragelement selber gebildet wird. Die stumpfe Spitze nimmt die beim Einschlagen des Instrumentes in den Zwischenwirbelraum auftretenden Kräfte auf. In diesem Zustand sind vorzugsweise die Klingen bzw. ist die Klinge in Längsrichtung gesehen hinter der stumpfen Spitze gelegen, sodass die Hebelmechanik der Klingen nicht durch die Kräfte beim Einschlagen belastet wird. Beim Aufweiten der Klingen können diese sich neben der radialen Bewegung in distaler Richtung so verlagern, dass sie in distaler Richtung über die stumpfe Spitze bzw. das stumpfe Ende vorstehen. Dies ermöglicht ein Abtragen auch im Bereich des distalen Endes des Schneidkopfes.

Die zweiten Anlenkungspunkte der drei Hebel, welche an einer Klinge, d.h. an derselben Klinge angelenkt sind, sind vorzugsweise auf einer Geraden gelegen, welche sich weiter bevorzugt parallel zur Verschieberichtung der Betätigungsstange, d.h. vorzugsweise parallel zur Längsachse des Instrumentenschaftes bzw. des Schneidkopfes erstreckt. Diese Anordnung begünstigt das gleichzeitige Verschwenken der drei Hebel in der Weise, dass die Schneidklinge radial nach außen bewegt wird. Insbesondere kann so eine parallelogrammartige Struktur, wie sie oben beschrieben wurde, geschaffen werden.

Gemäß einer weiteren bevorzugten Ausführungsform liegen die ersten Anlenkungspunkte der drei Hebel, welche an einer Klinge, d.h. derselben Klinge angelenkt sind, auf einer Geraden, welche sich parallel oder gewinkelt zur Verschieberichtung der Betätigungsstange erstreckt. Auch ist es möglich, dass sich ein Winkel zwischen dieser Geraden und der Verschieberichtung bzw. Längsachse während des Ausschwenkens bzw. Aufweitens ändert, um einen Lordosewinkel zu schaffen, wie er vorangehend beschrieben wurde. Im zusammengefalteten Ruhezustand, d.h. in dem Zustand, in dem die Klinge am Tragelement anliegt, erstreckt sich die Gerade, auf welcher die ersten Anlenkungspunkte liegen, vorzugsweise parallel zur Verschieberichtung bzw. Längsachse.

Beispielsweise, um einen gewünschten Lordosewinkel bereitstellen zu können, kann sich zumindest eine Außenseite der Klinge in zumindest einer Position gewinkelt zu einer Längsachse des Schneidkopfes bzw. des Instrumentes erstrecken. Auf diese Weise kann auch ein konstanter Lordosewinkel geschaffen werden, welcher nicht von der Auslenkung der Hebel abhängig ist. Die Hebel können dann so ausgestaltet werden, dass eine Parallelverschiebung der Schneidklinge zur Längsachse in radialer Richtung erfolgt und der Lordosewinkel, vorgegeben durch den Winkel der Klingenaußenseite, konstant ist.

Der Schneidkopf ist bevorzugt am distalen Ende eines Instrumentenschaftes angeordnet, wobei am proximalen Ende des Instrumentenschaftes eine Handhabe angeordnet ist, welche eine Betätigungseinrichtung aufweist, die mit der Betätigungsstange zu deren Verschieben gekoppelt ist. D.h. durch Betätigung der Betätigungseinrichtung kann die Betätigungsstande in gewünschter Weise linear verschoben werden. Die Betätigungseinrichtung kann beispielsweise ein Spindeltrieb sein, der ein auf einer Gewindespindel drehendes Drehrad aufweist. Dabei ist das Drehrad axial in der Handhabe fixiert, sodass durch Drehen des Betätigungsrades die Spindel in Längsrichtung vor- und zurückgeschoben werden kann. Diese Linearbewegung kann auf die Betätigungsstange übertragen werden. Dazu kann die Betätigungsstange fest mit einer Betätigungseinrichtung wie z.B. der Gewindespindel verbunden sein. Bevorzugt kann eine lösbare Kupplung vorgesehen sein. So können beispielsweise der Instrumentenschaft und die Betätigungsstange von der handhabe abnehmbar ausgebildet sein, um die Instrumentenschaft mit dem Schneidkopf als Artikel zum Einmalgebrauch ausbilden zu können, während die Handhabe wiederverwendet werden kann. In der Handhabe kann darüber hinaus eine Fixiereinrichtung zum lösbaren Fixieren des Instrumentenschaftes an der Handhabe vorgesehen sein. Dies kann beispielsweise eine Klemmschraube sein.

Gemäß einer weiteren bevorzugten Ausführungsform weist die Betätigungseinrichtung eine Skala auf, welche den Grad der Aufweitung des Schneidkopfes, d.h. das Maß der radialen Bewegung der Klinge bzw. der Klingen, anzeigt. Da die radiale Verlagerung der Klinge bzw. Klingen über das Auslenken der Hebel erfolgt, besteht möglicherweise kein linearer Zusammenhang zwischen einem Maß, um welches die Betätigungseinrichtung bewegt wird und der Auslenkung der Klinge. Dies kann bei der Ausgestaltung einer Skala an der Handhabe berücksichtigt sein, so kann die Skala nicht linear ausgebildet sein und das Maß anzeigen, um welches die Klingen bei der jeweiligen Stellung der Betätigungseinrichtung, beispielsweise der linearen Vorschubposition der Betätigungsstange, ausgelenkt sind.

Nachfolgend wird die Erfindung beispielhaft anhand der beigefügten Figuren beschrieben. In diesen zeigt:
- Fig. 1: eine Gesamtansicht des Abtragwerkzeuges für die Zwischenwirbelpräparation gemäß der Erfindung,
- Fig. 2: eine Detailansicht des Schneidkopfes des Abtragwerkzeuges gemäß Figur 1 im aufgeweiteten Zustand,
- Fig. 3: eine Draufsicht auf den Schneidkopf gemäß Figur 2,
- Fig. 4: eine teilweise geschnittene Ansicht des Schneidkopfes gemäß Figur 2,
- Fig. 5: eine Ansicht des Schneidkopfes gemäß Figur 4 im Ruhezustand,
- Fig. 6: eine alternative Ausführungsform eines erfindungsgemäßen Schneidkopfes im aufgeweiteten Zustand,
- Fig. 7: eine Detailansicht der Handhabe und
- Fig. 8: vergrößert den Ausschnitt VIII in Figur 7.

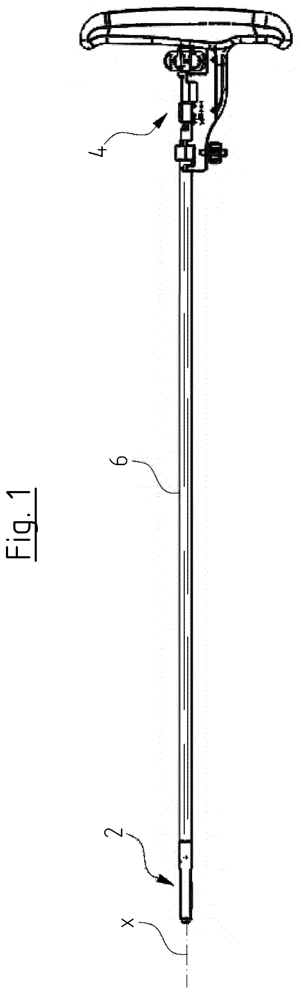

Figur 1 zeigt in einer Seitenansicht das Abtragwerkzeug als Gesamtinstrument. Das Gesamtinstrument besteht im Wesentlichen aus drei Teilen, einem Schneidkopf 2 am distalen Ende, einer Handhabe 4 am proximalen Ende und einem Instrumentenschaft 6, welcher sich von der Handhabe 4 zu dem Schneidkopf 2 in distaler Richtung erstreckt.

Der Schneidkopf 2 ist in diesem Ausführungsbeispiel fest am distalen Ende des Instrumentenschaftes angeordnet. Der Instrumentenschaft ist, wie nachfolgend beschrieben werden wird, lösbar mit der Handhabe 4 verbunden. So können Instrumentenschaft und Schneidkopf 2 beispielsweise als Einmalinstrument ausgebildet werden, während die Handhabe mehrfach verwendet werden kann, oder Handhabe 4 und Instrumentenschaft 6 können z.B. zur Reinigung getrennt werden.

Der Schneidkopf 2 weist zwei Klingen 8 auf, welche über Hebel an einem Tragelement 10 angelenkt sind. Das Tragelement 10 ist am distalen Ende des Instrumentenschaftes 6 befestigt oder kann auch ganz oder in Teilen einstückig mit diesem ausgebildet sein. Das distale Ende des Tragelementes 10 ist als stumpfe Spitze 12 ausgebildet, welche beim Einschieben bzw. Einschlagen des Instrumentes die auftretenden Kräfte aufnimmt.

Jede der Klingen 8 ist über drei Hebel mit dem Tragelement 10 verbunden, einem ersten Hebel 14, einem zweiten Hebel 16 und einem dritten Hebel 18. Der erste Hebel ist distalseitig gelegen, der dritte Hebel proximalseitig und der zweite Hebel 16 dazwischen. Die Anordnung der beiden Klingen 8 mit ihren Hebeln 14, 16 und 18 ist spiegelsymmetrisch zu der Längsachse X des Instrumentes bzw. des Schneidkopfes 2, welche sich vom proximalen zum distalen Ende erstreckt.

Die Hebel 14, 16 und 18 sind mit den Klingen 8 jeweils über erste Anlenkungspunkte 20, welche Schwenkachsen bilden oder als Schwenkachsen ausgebildet sind, gelenkig verbunden. Um die Anlenkungspunkte 20 können die Hebel 14, 16, 18 relativ zu den Klingen 8 verschwenken. An ihrem entgegengesetzten Längsende sind der erste Hebel 14 und zweite Hebel 16 jeweils über einen zweiten Anlenkungspunkt 22 an dem Tragelement 10 schwenkbar befestigt. Die dritten Hebel 18 sind an ihren den ersten Anlenkungspunkten 20 entgegengesetzten Enden jeweils über einen zweiten Anlenkungspunkt 24 schwenkbar mit einer Betätigungsstange 26 verbunden. Die Betätigungsstange erstreckt sich in Richtung der Längsachse X im Inneren des Instrumentenschaftes 6 und ist relativ zu diesem und relativ zu dem Tragelement 10 in der Längsrichtung X vor und zurück verschiebbar. Die durch die Anlenkungspunkte 20, 22 und 24 definierten Schwenkachsen erstrecken sich normal zu der Längsrichtung X und damit der Bewegungsrichtung der Betätigungsstange 26.

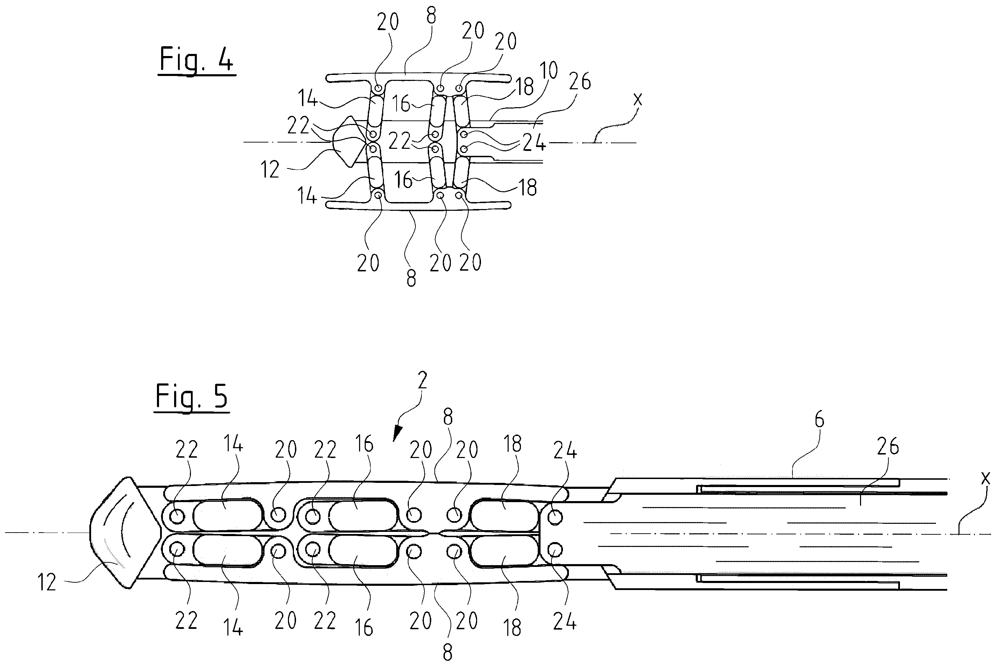

In dem in Figuren 4 und 5 gezeigten Beispiel sind die ersten Hebel 14 und zweiten Hebel 16 gleich lang und deren erste Anlenkungspunkte 20 sind in Richtung der Längsachse X gleich weit beabstandet wie die zweiten Anlenkungspunkte 22. So bilden die ersten und zweiten Hebel 14, 16 mit ihrer jeweils zugehörigen Klinge 8 eine Parallelogrammanordnung. Figur 4 zeigt die Klingen 8 in ihrem ausgefahrenen Zustand, in welchem sie in radialer Richtung maximal von der Längsachse X beabstandet sind, d.h. in diesem Zustand weist der Schneidkopf seinen größten Durchmesser auf. Figur 5 zeigt den Ruhezustand, in welchem die Schneidklingen 8 im Wesentlichen an dem Tragelement 10 anliegen. Dabei liegen die Klingen 8 proximalseitig hinter der Spitze 12, sodass die Klingen 8 beim Einschieben bzw. Einschlagen durch die stumpfe Spitze 12 geschützt werden. In dem ausgefahrenen Zustand, welcher in Figur 4 gezeigt ist, sind die Klingen 8 nicht nur in radialer Richtung bewegt, sondern durch das parallele Verschwenken mit den Hebeln auch distalwärts bewegt, sodass die distalen Enden der Klingen 8 über die Spitze 12 über stehen. So kann der Schneidkopf bis zu seinem distalen Ende hin schneidend wirken.

Um die Klingen 8 aus dem in Figur 5 gezeigten Ruhezustand radial nach außen zu bewegen, verschwenken die ersten und zweiten Hebel 14 und 16 in derselben Richtung um ihre zweiten Anlenkungspunkte 22 bzw. zweiten Schwenkachsen 22 in der Weise, dass sich die ersten Anlenkungspunkte 20 der ersten Hebel 14 und zweiten Hebel 16 im Bogen distalwärts zu der Spitze 12 hin bewegen und so die Klingen 8 radial nach außen und gleichzeitig distalwärts bewegen, wobei die Klingen 8 sich konstant parallel zu der Längsachse X erstrecken. Veranlasst wird diese Bewegung, indem die Betätigungsstange 26 distalwärts geschoben wird. Dabei verlagern sich die zweiten Anlenkungspunkte 24 der dritten Hebel 18 distalwärts, wodurch die dritten Hebel 18 um ihre zweiten Anlenkungspunkte 24 verschwenken und so die ersten Anlenkungspunkte 20, über welche sie an den Klingen 8 befestigt sind, radial nach außen drücken. Die dritten Hebel 18 der beiden Klingen 8 bilden dabei mit der Betätigungsstange 26 eine Art Kniehebel, bei welchem die Betätigungsstange 26 zentral am "Knie" angreift. So kann über eine vergleichsweise kleine Kraft in Richtung der Längsrichtung X eine große Kraft in radialer Richtung quer zu der Längsachse X erzielt werden.

Die dritten Hebel 18 verschwenken bei ihrer Bewegung in entgegengesetzter Richtung zu den zweiten Hebeln 16 an der jeweiligen Klinge 8. Dies bewirkt, dass die dritten Hebel 18 sich nicht parallel zu den zweiten Hebeln 16 erstrecken, sondern stets gewinkelt zu diesen gerichtet sind, sodass der zweite Hebel 16 und der dritte Hebel 18 an jeder der Klingen 8 V-förmig bzw. trapezförmig zueinander gerichtet sind, wobei ihre ersten Anlenkungspunkte 20 parallel zur Längsachse X näher beieinander gelegen sind als die zweiten Anlenkungspunkte 22 und 24. Durch diese Ausgestaltung wird verhindert, dass sich die Winkellagen der Klingen 8 relativ zu der Längsachse X unbeabsichtigt ändern können. Während sich die ersten und zweiten Hebel 16 und 18 ausgehend von dem Tragelement 10 zu ihren ersten Anlenkungspunkten 20 hin schräg zur Längsachse X in proximaler Richtung erstrecken, erstrecken sich die dritten Hebel 18 ausgehend von der Betätigungsstange 26 zu ihren ersten Anlenkungspunkten 20 schräg zur Längsachse X in distaler Richtung. Je nachdem, wie weit die Betätigungsstange 26 in distaler Richtung vorgeschoben wird, bewegen sich die Klingen 8 ausgehend von der Längsachse X unterschiedlich weit radial nach außen. So kann der Durchmesser bzw. der Abstand der Klingen 8 quer zur Längsachse X variiert und eingestellt werden.

In dem in Figur 5 gezeigten Ruhezustand erstrecken sich die Hebel 14, 16 und 18 jeder der beiden Klingen 8 im Wesentlichen in einer Linie, nur mit sehr geringen Winkeln zur Längsachse X. Um sicherzustellen, dass die Hebel ausgehend von dieser Ruhelage beim Vorschub der Betätigungsstange 26 verschwenken, ist es wichtig, dass die Kraft, welche über die Betätigungsstange 26 auf die Hebel 18 eingeleitet wird, sich nicht genau in Richtung einer Gerade durch den ersten Anlenkungspunkt 20 und der zweite Anlenkungspunkt 24 der dritten Hebel 18 erstreckt. D.h. die Verbindungslinie, welche die Mittelpunkte des ersten Anlenkungspunktes 20 und des Anlenkungspunktes 24 verbindet, erstreckt sich in der Ruhelage, welche in Figur 5 gezeigt ist, gewinkelt zu der Längsachse X. Die entsprechend definierten Längsachsen der ersten Hebel 14 und zweiten Hebel 16, welche sich durch die Mittelpunkte der ersten Anlenkungspunkte 20 und zugehörigen Anlenkungspunkte 22 erstrecken, erstrecken sich in einem spitzen Winkel zur Längsachse X. In dem hier gezeigten Beispiel liegen alle ersten Anlenkungspunkte 20 an einer Klinge 8 auf einer Geraden, welche parallel zur Längsachse X verläuft. Entsprechend liegen die zugehörigen zweiten Anlenkungspunkte 22 und 24 jeder der Klingen 8 ebenfalls im Wesentlichen auf einer sich parallel zur Längsachse X erstreckenden Geraden. Um die Schwenkbewegung der dritten Hebel 18 entgegengesetzt zu der Schwenkbewegung der ersten Hebel 14 und zweiten Hebel 16 zu ermöglichen, sind die dritten Hebel 18 an der zugehörigen Klinge 8 jeweils so angeordnet, dass der erste Anlenkungspunkt 20 weiter distalwärts gelegen ist als der zugehörige zweite Anlenkungspunkt 24. Bei dem ersten Hebel 14 und dem zweiten Hebel 16 ist dies umgekehrt. Dort liegt jeweils der zweiter Anlenkungspunkt 22 weiter distalseitig als der erste Anlenkungspunkt 20.

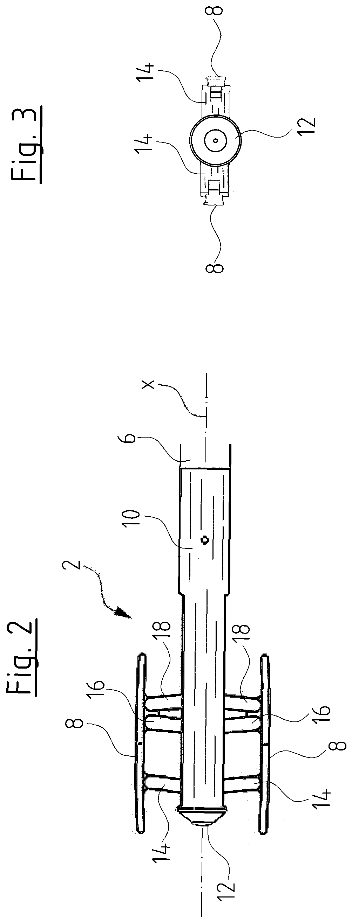

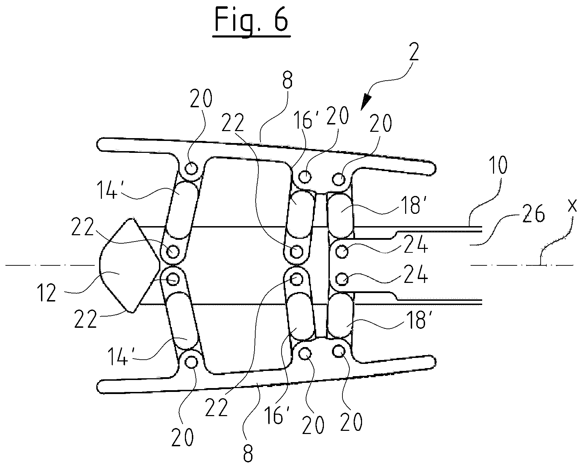

In den in Figuren 2 bis 5 gezeigten Beispielen erstrecken sich die Außenseiten der Klingen 8 im Wesentlichen parallel zur Längsachse X, d.h. es ist im Wesentlichen kein Lordosewinkel zwischen den Klingen 8 vorgesehen. Eine erste Möglichkeit, wie ein Lordosewinkel ausgebildet werden könnte, wäre, die Außenseiten der Klingen 8 schräg zu der Geraden, welche durch die ersten Anlenkungspunkte 20 verläuft, auszubilden. Eine zweite Alternative ist in Figur 6 gezeigt. Bei dem Ausführungsbeispiel gemäß Figur 6 wird ein Lordosewinkel dadurch realisiert, dass die Hebel 14', 16' und 18', welche die Klingen 8 an dem Tragelement 10 anlenken, nicht gleich lang ausgebildet sind. In dem hier gezeigten Beispiel weisen die ersten Hebel 14' zwischen ihren ersten Anlenkungspunkten 20 und den Anlenkungspunkten 22 jeweils eine größere Länge auf, als die zweiten Hebel 16'. Die dritten Hebel 18' sind wiederrum kürzer ausgebildet als die zweiten Hebel 16'. Dies bewirkt, dass die ersten Hebel 14' und 16' an jeder der Klingen 8 nicht mehr exakt parallel angeordnet sind und keine exakte Parallelogrammstruktur bilden. Dies führt dazu, dass beim Ausfahren bzw. Ausschwenken der Klingen 8 aus einer Ruheposition entsprechend Figur 5 die Klingen 8 beim Aufweiten zunehmend gewinkelt zur Längsachse X ausrichten. D.h. hier wird kein konstanter Lordosewinkel geschaffen, sondern der Lordosewinkel nimmt im Wesentlich mit dem Aufweiten bzw. Ausfahren der Klingen 8 in radialer Richtung zu, im gezeigten Beispiel von etwa 0 Grad bis 9 Grad.

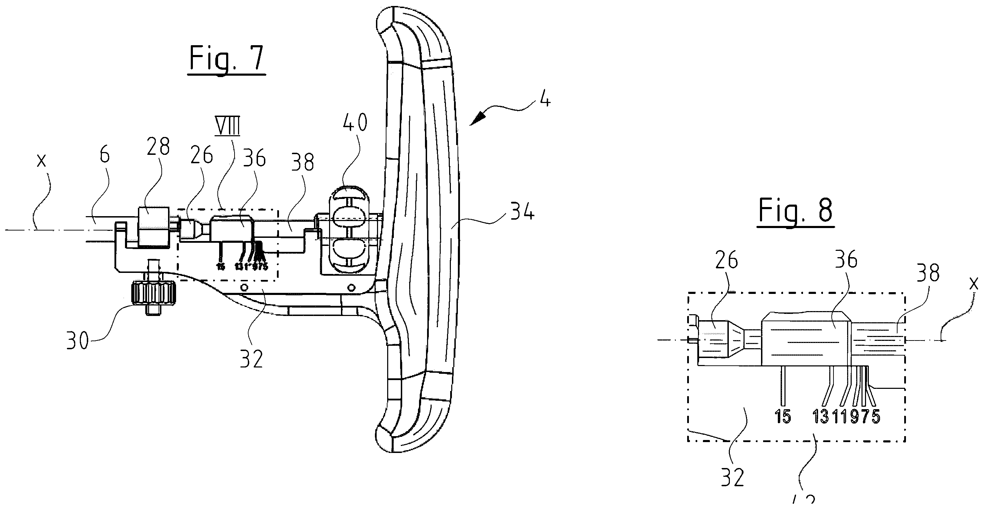

Die Figuren 7 und 8 zeigen eine Detailansicht der Handhabe 4. Der Instrumentenschaft 6 bzw. dessen Außenschaft ist über einen Haltearm 28 an der Handhabe 4 klemmend fixiert. Zur Klemmung kann der Haltearm 28 über eine Rändelschraube 30 relativ zu einer Griffbasis 32 verstellt werden, sodass der Instrumentenschaft 6 zwischen dem Haltearm 28 und der Griffbasis 32 geklemmt werden kann. Die Betätigungsstange 26 ist an ihrem proximalen Ende durch einen Formschluss mit einem Schieber 36 verbunden, welcher gleitend in Richtung der Längsachse X an der Griffbasis 32 geführt ist. An dem Schieber 36 ist eine Gewindestange 38 angebracht, welche sich in Richtung der Längsachse X erstreckt und in eine Betätigungseinrichtung in Form eines Verstellrades 40 eingreift. Das Verstellrad 40 ist in axialer Richtung in der Griffbasis 32 gesichert, sodass bei Drehung des Verstellrades 40 in dessen Inneren über einen Gewindeeingriff die Gewindestange 38 in Längsrichtung X verschoben wird. Über die Gewindestange 38 wird die gekoppelte Betätigungsstange 26 ebenfalls in axialer Richtung bzw. Längsrichtung X verschoben, um die Klingen 8 in der beschriebenen Weise auseinander oder aufeinander zu bewegen zu können.

Um an der Handhabe 4 die Stellung der Klingen 8 erkennen zu können, ist auf der Griffbasis 32 eine Skala 42 ausgebildet, welche in Zusammenwirkung mit dem Schieber 36 das Maß der radialen Auslenkung bzw. Aufweitung der Klingen 8 anzeigt. Da aufgrund der Hebelmechanik die Verschiebung der Betätigungsstange 26 nicht linear in eine radiale Bewegung der Klingen 8 umgesetzt wird, ist die Skala 42 nicht linear ausgebildet. Mit zunehmender Verlagerung des Schiebers 36 in distaler Richtung nehmen die Skalenabstände zu. Aus der Relativposition des Schiebers 36 zu der Skala 42 kann das Maß der radialen Aufweitung, d.h. der Durchmesser des Schneidkopfes zwischen den Klingen 8 an der Handhabe 4 abgelesen werden.

### Bezugszeichenliste

- 2: Schneidkopf
- 4: Handhabe
- 6: Instrumentenschaft
- 8: Klingen
- 10: Tragelement
- 12: Spitze
- 14, 14': erster Hebel
- 16, 16': zweiter Hebel
- 18, 18': dritter Hebel
- 20: erster Anlenkungspunkt
- 22, 24: zweiter Alenkungspunkt
- 26: Betätigungsstange
- 28: Haltearm
- 30: Rändelschraube
- 32: Griffbasis
- 34: Griffstück
- 36: Schieber
- 38: Gewindestange
- 40: Verstellrad
- 42: Skala
- X: Längsachse

## Patentansprüche

1. Zwischenwirbelpräparationswerkzeug mit einem Schneidkopf (2), der zumindest eine bewegliche Klinge (8) aufweist, welche von zumindest drei schwenkbaren Hebeln gehalten ist, wobei die Hebel (14, 16, 18) jeweils starr ausgebildet sind,
ein erster (14) und ein zweiter (16) Hebel jeweils an einem ersten Anlenkungspunkt (20) an der Klinge (8) und an einem beabstandeten zweiten Anlenkungspunkt (22) an einem Tragelement (10) des Schneidkopfes (2) angelenkt sind,
ein dritter Hebel (18) an einem ersten Anlenkungspunkt (20) an der Klinge (8) und an einem beabstandeten zweiten Anlenkungspunkt (24) an einer relativ zu dem Tragelement (10) verschiebbaren Betätigungsstange (26) angelenkt ist, und wobei
die Anlenkungspunkte (20, 22, 24) als Schwenkgelenke mit zueinander parallelen Schwenkachsen ausgebildet sind.

2. Zwischenwirbelpräparationswerkzeug nach Anspruch 1, bei welchem der Schneidkopf (2) zwei bewegliche Klingen (8) aufweist, welche jeweils von drei schwenkbaren Hebeln (14, 16, 18) gehalten sind, wobei
die Hebel (14, 16, 18) jeweils starr ausgebildet sind,
ein erster (14) und ein zweiter (16) Hebel jeweils an einem ersten Anlenkungspunkt (20) an der zugehörigen Klinge (8) und an einem beabstandeten zweiten Anlenkungspunkt (22) an dem Tragelement (10) angelenkt sind,
ein dritter Hebel (18) an einem ersten Anlenkungspunkt (20) an der zugehörigen Klinge (8) und an einem beabstandeten zweiten Anlenkungspunkt (24) an der relativ zu dem Tragelement (10) verschiebbaren Betätigungsstange (26) angelenkt ist, und wobei die Anlenkungspunkte (20, 22, 24) als Schwenkgelenke mit zueinander parallelen Schwenkachsen ausgebildet sind.

3. Zwischenwirbelpräparationswerkzeug nach Anspruch 2, bei welchem die zwei Klingen (8) an entgegengesetzten Seiten einer Längsachse (X) des Schneidkopfes (2), vorzugsweise symmetrisch zueinander, angeordnet sind.

4. Zwischenwirbelpräparationswerkzeug nach einem der Ansprüche 1 bis 3, bei welchem die zweiten Anlenkungspunkte (22, 24) der drei Hebel (14, 16, 18) zum Halten einer Klinge (8) in einer Richtung quer zu den Schwenkachsen voneinander beabstandet sind.

5. Zwischenwirbelpräparationswerkzeug nach einem der vorangehenden Ansprüche, bei welchem am Schneidkopf (2) der erste Hebel (14) einen distalseitigen Hebel und der dritte Hebel (18) einen proximalseitigen Hebel bilden und der zweite Hebel (16) zwischen dem ersten (14) und dem dritten (18) Hebel angeordnet ist.

6. Zwischenwirbelpräparationswerkzeug nach einem der vorangehenden Ansprüche, bei welchem an einer Klinge (8) die ersten Anlenkungspunkte (20) des ersten (14) und des zweiten (16) Hebels und vorzugsweise die ersten Anlenkungspunkte (20) des ersten (14) , des zweiten (16) und des dritten (18) Hebels in einer Richtung quer zu den Schwenkachsen voneinander beabstandet sind.

7. Zwischenwirbelpräparationswerkzeug nach einem der vorangehenden Ansprüche, bei welchem die Hebel (14, 16, 18) und deren Anlenkungspunkte (20, 22, 24) so gelegen und dimensioniert sind, dass durch Verschwenken der Hebel (14, 16, 18) um ihre zweiten Anlenkungspunkte (22, 24) die zugehörige Klinge (8) von einer ersten dem Tragelement (10) nahen in zumindest eine zweite von dem Tragelement (10) weiter beabstandete Position bewegbar ist.

8. Zwischenwirbelpräparationswerkzeug nach einem der vorangehenden Ansprüche, bei welchem der erste (14) und der zweite (16) Hebel so angelenkt sind, dass sie zum Bewegen der verbundenen Klinge (8) in derselben Drehrichtung verschwenken.

9. Zwischenwirbelpräparationswerkzeug nach einem der vorangehenden Ansprüche, bei welchem der dritte Hebel (18) so angelenkt ist, dass er zum Bewegen der verbundenen Klinge (8) in einer Drehrichtung verschwenkt, welche der Drehrichtung des ersten (14) und/oder zweiten (16) Hebels bei deren gleichzeitigen Verschwenken entgegengesetzt ist.

10. Zwischenwirbelpräparationswerkzeug nach einem der vorangehenden Ansprüche, bei welchem der erste (14) und der zweite (16) Hebel, welche an einer Klinge (8) angelenkt sind, zwischen ihren ersten (20) und zweiten (22) Anlenkungspunkten dieselbe Länge aufweisen.

11. Zwischenwirbelpräparationswerkzeug nach einem der vorangehenden Ansprüche, bei welchem der zweite (16) und der dritte (18) Hebel, welche an einer Klinge (8) angelenkt sind, zwischen ihren ersten (20) und zweiten (22, 24) Anlenkungspunkten dieselbe Länge aufweisen.

12. Zwischenwirbelpräparationswerkzeug nach einem der vorangehenden Ansprüche, bei welchem der erste Hebel (14') und der zweite Hebel (16'), welche an einer Klinge (8) angelenkt sind, zwischen ihren ersten (20) und zweiten (22) Anlenkungspunkten unterschiedliche Längen aufweisen, wobei vorzugsweise der erste Hebel (14'), welcher einen distalseitigen Hebel am Schneidkopf (2) bildet, länger als der zweite Hebel (16') ausgebildet ist.

13. Zwischenwirbelpräparationswerkzeug nach einem der vorangehenden Ansprüche, bei welchem der zweite Hebel (16') und der dritte Hebel (18'), welche an einer Klinge (8) angelenkt sind, zwischen ihren ersten (20) und zweiten (22, 24) Anlenkungspunkten unterschiedliche Längen aufweisen, wobei vorzugsweise der zweite Hebel (16') länger als der dritte Hebel (18') ausgebildet ist.

14. Zwischenwirbelpräparationswerkzeug nach einem der vorangehenden Ansprüche, bei welchem der Schneidkopf (2) an einem distalen Ende eines Instrumentenschaftes (6) gelegen ist und die Betätigungsstange (26) in Längsrichtung (X) des Instrumentenschaftes (6) verschiebbar an oder in dem Instrumentenschaft (6) geführt ist.

15. Zwischenwirbelpräparationswerkzeug nach einem der vorangehenden Ansprüche, bei welchem die zweiten Anlenkungspunkte (22, 24) der drei Hebel (14, 16, 18), welche an einer Klinge (8) angelenkt sind, auf einer Geraden liegen, welche sich vorzugsweise parallel zur Verschieberichtung (X) der Betätigungsstange (26) erstreckt.

16. Zwischenwirbelpräparationswerkzeug nach einem der vorangehenden Ansprüche, bei welchem die ersten Anlenkungspunkte (20) der drei Hebel (14, 16, 18), welche an einer Klinge (8) angelenkt sind, auf einer Gerade liegen, welche sich parallel oder gewinkelt zur Verschieberichtung (X) der Betätigungsstange (26) erstreckt.

17. Zwischenwirbelpräparationswerkzeug nach einem der vorangehenden Ansprüche, bei welchem sich zumindest eine Außenseite der Klinge (8) in zumindest einer Position gewinkelt zu einer Längsachse (X) des Schneidkopfes (2) erstreckt.

18. Zwischenwirbelpräparationswerkzeug nach einem der vorangehenden Ansprüche, bei welchem der Schneidkopf (2) am distalen Ende eines Instrumentenschaftes (6) angeordnet ist und am proximalen Ende des Instrumentenschaftes (6) eine Handhabe (4) angeordnet ist, welche eine Betätigungseinrichtung (40) aufweist, die mit der Betätigungsstange (26) zu deren Verschieben gekoppelt ist.

## Claims

1. An intervertebral preparation tool comprising a cutting head (2), which has at least one movable blade (8) which is held by at least three pivotable levers, wherein the levers (14, 16, 18) are each rigid,
a first (14) and a second (16) lever are each articulated at a first articulation point (20) on the blade (8) and at a distanced second articulation point (22) on a carrier element (10) of the cutting head (2),
a third lever (18) is articulated at a first articulation point (20) on the blade (8) and at a distanced second articulation point (24) on an actuation rod (26) which is displaceable relative to the carrier element (10), and wherein
the articulation points (20, 22, 24) are configured as pivot joints with pivot axes which are parallel to one another.

2. The intervertebral preparation tool according to claim 1, in which the cutting head (2) has two movable blades (8) which are each held by three pivotable levers (14, 16, 18), wherein
the levers (14, 16, 18) are each rigid,
a first (14) and a second (16) lever are each articulated at a first articulation point (20) on the associated blade (8) and at a distanced second articulation point (22) on the carrier element (10), a third lever (18) is articulated at a first articulation point (20) on the associated blade (8) and at a distanced second articulation point (24) on the actuation rod (26) which is displaceable relative to the carrier element (10), and wherein
the articulation points (20, 22, 24) are configured as pivot joints with pivot axes which are parallel to one another.

3. The intervertebral preparation tool according to claim 2, in which the two blades (8) are arranged at opposite sides of a longitudinal axis (X) of the cutting head (2), preferably symmetrically to one another.

4. The intervertebral preparation tool according to any one of claims 1 to 3, in which the second articulation points (22, 24) of the three levers (14, 16, 18) for holding a blade (8) are distanced from one another in a direction transverse to the pivot axes.

5. The intervertebral preparation tool according to any one of the preceding claims, in which on the cutting head (2) the first lever (14) forms a distal-side lever and the third lever (18) forms a proximal-side lever and the second lever (16) is arranged between the first (14) and the third (18) lever.

6. The intervertebral preparation tool according to any one of the preceding claims, in which on a blade (8) the first articulation points (20) of the first (14) and the second (16) lever and preferably the first articulation points (20) of the first (14), the second (16) and the third (18) lever are distanced from one another in a direction transverse to the pivot axes.

7. The intervertebral preparation tool according to any one of the preceding claims, in which the levers (14, 16, 18) and their articulation points (20, 22, 24) are situated and dimensioned such that by way of pivoting the levers (14, 16, 18) about their second articulation points (22, 24) the associated blade (8) is movable from a first position which is close to the carrier element (10) into at least one second position which is distanced further from the carrier element (10).

8. The intervertebral preparation tool according to any one of the preceding claims, in which the first (14) and the second (16) levers are articulated such that for moving the connected blade (8) the first and the second levers pivot in the same rotation direction.

9. The intervertebral preparation tool according to any one of the preceding claims, in which the third lever (18) is articulated such that for moving the connected blade (8) the third lever pivots in a rotation direction which is opposite to the rotation direction of the first (14) and/or second (16) lever in their simultaneous pivoting.

10. The intervertebral preparation tool according to any one of the preceding claims, in which the first (14) and the second (16) lever which are articulated on a blade (8) have the same length between their first (20) and second (22) articulation points.

11. The intervertebral preparation tool according to any one of the preceding claims, in which the second (16) and the third (18) lever which are articulated on a blade (8) have the same length between their first (20) and second (22, 24) articulation points.

12. An intervertebral preparation tool according to any one of the preceding claims, in which the first lever (14') and the second lever (16') which are articulated on a blade (8) have different lengths between their first (20) and second (22) articulation points, wherein the first lever (14'), which forms a distal-side lever on the cutting head (2), is longer than the second lever (16').

13. The intervertebral preparation tool according to any one of the preceding claims, in which the second lever (16') and the third lever (18') which are articulated on a blade (8) have different lengths between their first (20) and second (22, 24) articulation points, wherein the second lever (16') is preferably longer than the third lever (18').

14. The intervertebral preparation tool according to any one of the preceding claims, in which the cutting head (2) is situated at a distal end of an instrument shank (6) and the actuation rod (26) is displaceably guided in the longitudinal direction (X) of the instrument shank (6) on or in the instrument shank (6).

15. The intervertebral preparation tool according to any one of the preceding claims, in which the second articulation points (22, 24) of the three levers (14, 16, 18) which are articulated on a blade (8) lie on a straight line which preferably extends parallel to a displacement direction (X) of the actuation rod (26).

16. The intervertebral preparation tool according to any one of the preceding claims, in which the first articulation points (20) of the three levers (14, 16, 18) which are articulated on a blade (8) lie on a straight line which extends parallel or at an angle to the displacement direction (X) of the actuation rod (26).

17. The intervertebral preparation tool according to any one of the preceding claims, in which at least one outer side of the blade (8) in at least one position extends at an angle to a longitudinal axis (X) of the cutting head (2).

18. The intervertebral preparation tool according to any one of the preceding claims, in which the cutting head (2) is arranged at the distal end of an instrument shank (6) and a handle (4) is arranged at the proximal end of the instrument shank (6), which handle has an actuation device (40) which is coupled to the actuation rod (26) for displacement thereof.

## Revendications

1. Outil de préparation intervertébrale comprenant une tête de coupe (2) qui présente au moins une lame mobile (8), laquelle est maintenue par au moins trois leviers pivotants, dans lequel les leviers (14, 16, 18) sont respectivement rigides,
un premier (14) et un deuxième (16) levier sont articulés respectivement sur un premier point d'articulation (20) sur la lame (8) et sur un deuxième point d'articulation (22) distant sur un élément porteur (10) de la tête de coupe (2),
un troisième levier (18) est articulé sur un premier point d'articulation (20) sur la lame (8) et sur un deuxième point d'articulation (24) distant sur une barre d'actionnement (26) mobile par rapport à l'élément porteur (10), et dans lequel les points d'articulation (20, 22, 24) sont conçus en tant qu'articulations pivotantes avec des axes pivotants parallèles entre eux.

2. Outil de préparation intervertébrale selon la revendication 1, dans lequel la tête de coupe (2) présente deux lames mobiles (8), lesquelles sont respectivement maintenues par trois leviers pivotants (14, 16, 18), dans lequel
les leviers (14, 16, 18) sont respectivement rigides,
un premier (14) et un deuxième (16) levier sont articulés respectivement sur un premier point d'articulation (20) sur la lame (8) correspondante et sur un deuxième point d'articulation (22) distant sur l'élément porteur (10),
un troisième levier (18) est articulé sur un premier point d'articulation (20) sur la lame (8) et sur un deuxième point d'articulation (24) distant sur la barre d'actionnement (26) mobile par rapport à l'élément porteur (10), et dans lequel
les points d'articulation (20, 22, 24) sont conçus en tant qu'articulations pivotantes avec des axes pivotants parallèles entre eux.

3. Outil de préparation intervertébrale selon la revendication 2, dans lequel les deux lames (8) sont disposées sur des côtés opposés d'un axe longitudinal (X) de la tête de coupe (2), de préférence symétriques entre elles.

4. Outil de préparation intervertébrale selon l'une des revendications 1 à 3, dans lequel les deux points d'articulation (22, 24) des trois leviers (14, 16, 18) sont distants l'un de l'autre pour maintenir une lame (8) dans un sens transversal aux axes pivotants.

5. Outil de préparation intervertébrale selon l'une des revendications précédentes, dans lequel sur la tête de coupe (2), le premier levier (14) forme un levier côté distal et le troisième levier (18) forme un levier côté proximal, et le deuxième levier (16) est disposé entre le premier (14) et le troisième (18) levier.

6. Outil de préparation intervertébrale selon l'une des revendications précédentes, dans lequel sur une lame (8), les premiers points d'articulation (20) du premier (14) et du deuxième (16) levier et de préférence les premiers points d'articulation (20) du premier (14), du deuxième (16) et du troisième (18) leviers sont distants entre eux dans un sens transversal aux axes pivotants.

7. Outil de préparation intervertébrale selon l'une des revendications précédentes, dans lequel les leviers (14, 16, 18) et leurs points d'articulation (20, 22, 24) sont ainsi situés et dimensionnés que par un pivotement des leviers (14, 16, 18) sur leurs deuxièmes points d'articulation (22, 24), la lame (8) correspondante est mobile d'une première position proche de l'élément porteur (10) à une deuxième plus éloignée de l'élément porteur (10).

8. Outil de préparation intervertébrale selon l'une des revendications précédentes, dans lequel le premier (14) et le deuxième (16) levier sont ainsi articulés qu'ils pivotent dans le même sens de rotation pour déplacer la lame (8) reliée.

9. Outil de préparation intervertébrale selon l'une des revendications précédentes, dans lequel le troisième levier (18) est ainsi articulé qu'il pivote dans un sens de rotation pour déplacer la lame (8) reliée qui est opposé au sens de rotation du premier (14) et/ou du deuxième (16) levier lors de leur pivotement simultané.

10. Outil de préparation intervertébrale selon l'une des revendications précédentes, dans lequel le premier (14) et le deuxième (16) levier qui sont articulés sur une lame (8), présentent la même longueur entre leurs premier (20) et deuxième (22) points d'articulation.

11. Outil de préparation intervertébrale selon l'une des revendications précédentes, dans lequel le deuxième (16) et le troisième (18) leviers qui sont articulés sur une lame (8), présentent la même longueur entre leurs premier (20) et deuxièmes (22, 24) points d'articulation.

12. Outil de préparation intervertébrale selon l'une des revendications précédentes, dans lequel le premier levier (14') et le deuxième levier (16') qui sont articulés sur une lame (8), présentent différentes longueurs entre leurs premier (20) et deuxièmes (22) points d'articulation, dans lequel de préférence le premier levier (14') qui forme un levier côté distal sur la tête de coupe (2), est plus long que le deuxième levier (16').

13. Outil de préparation intervertébrale selon l'une des revendications précédentes, dans lequel le deuxième levier (16') et le troisième levier (18') qui sont articulés sur une lame (8), présentent différentes longueurs entre leurs premier (20) et deuxièmes (22, 24) points d'articulation, dans lequel de préférence le deuxième levier (16') est plus long que le troisième levier (18').

14. Outil de préparation intervertébrale selon l'une des revendications précédentes, dans lequel la tête de coupe (2) est située à une extrémité distale d'une tige d'instrument (6) et la barre d'actionnement (26) dans le sens longitudinal (X) de la tige d'instrument (6) est dirigée mobile sur ou dans la tige d'instrument (6).

15. Outil de préparation intervertébrale selon l'une des revendications précédentes, dans lequel les deuxièmes points d'articulation (22, 24) des trois leviers (14, 16, 18) qui sont articulés sur une lame (8), reposent sur une droite qui s'étend de préférence parallèlement au sens de déplacement (X) de la barre d'actionnement (26).

16. Outil de préparation intervertébrale selon l'une des revendications précédentes, dans lequel les premiers points d'articulation (20) des trois leviers (14, 16, 18) qui sont articulés sur une lame (8), reposent sur une droite qui s'étend de préférence parallèlement ou en angle au sens de déplacement (X) de la barre d'actionnement (26).

17. Outil de préparation intervertébrale selon l'une des revendications précédentes, dans lequel au moins un côté extérieur de la lame (8) s'étend dans au moins une position en angle par rapport à un axe longitudinal (X) de la tête de coupe (2).

18. Outil de préparation intervertébrale selon l'une des revendications précédentes, dans lequel la tête de coupe (2) est disposée à l'extrémité distale d'une tige d'instrument (6), et à l'extrémité proximale de la tige d'instrument (6) est disposée une poignée (4) qui présente un dispositif d'actionnement (40) qui est couplé à la barre d'actionnement (26) pour la déplacer.
